# EUROPEAN PATENT APPLICATION

(11) **EP 3 730 170 A1**
(43) Date of publication of application: **28.10.2020**
(21) Application number: 18890043.5
(22) Date of filing: 18.12.2018
(51) Int. Cl.: A61M 5/28, A61F 2/00, B65B 3/00, A61K 9/00, A61K 31/167, A61K 8/73

(54) **METHOD FOR MANUFACTURING PRE-FILLING TYPE SYRINGE COMPRISING LOCAL ANESTHETIC AND HYALURONIC ACID HYDROGEL**

(30) Priority: 19.12.2017 KR 20170175282
(71) Applicant: Dae Hwa Pharma. Co., Ltd, Hoengseong-gun, Gangwon-do 25228 (KR)
(72) Inventor: LEE, Ji-Yeon, Seongnam-si Gyeonggi-do 13175 (KR); SONG, In-beom, Suwon-si Gyeonggi-do 16456 (KR); SON, Minhee, Seongnam-si, Gyeonggi-do 13491 (KR); LEE, In-Hyun, Gwangju 61260 (KR)
(74) Representative: Arnold & Siedsma
(86) International application number: PCT/KR2018/016132
(87) International publication number: WO 2019/124938

(57) **Abstract**

**[Summary]**

The present invention relates to a method for manufacturing a pre-filling type syringe comprising a hyaluronic acid hydrogel containing a local anesthetic and a pre-filling type syringe manufactured thereby and, more particularly, to a pre-filling type syringe wherein a local anesthetic, such as lidocaine, or a pharmaceutically acceptable salt thereof is allowed to be adsorbed on an inner wall of a syringe and then coated onto a hydrogel surface, thereby exhibiting an excellent initial release of the local anesthetic or pharmaceutically acceptable salt thereof during the operation or injection, so that the syringe of the present invention can, even when distributed and stored for a long period of time, exhibit an anesthetic effect promptly during injection of a dermal filler or the like, and a method for manufacturing the same.

## Description

### [Technical Field]

### CROSS-REFERENCE TO RELATED APPLICATION(S)

This application claims the benefit of Korean Patent Application No. 10-2017-0175282 filed on December 19, 2017 with the Korean Intellectual Property Office, the disclosure of which is herein incorporated by reference in its entirety.

The present invention relates to a method for manufacturing a pre-filled syringe comprising a hyaluronic acid hydrogel and a local anesthetic and, more particularly, to a pre-filled syringe wherein a local anesthetic is allowed to be adsorbed on an inner wall of a syringe and then coated onto a hydrogel surface, thereby exhibiting an excellent initial release of lidocaine during the operation or injection, and a method for manufacturing the same.

### [Background Art]

The most common esthetic signs of facial aging include visibility of skin wrinkles, deep nasolabial folds, glabellar lines, marionette lines, buccal commissures, and perioral wrinkles. These aging changes are often treated by the injection of dermal fillers to increase the tissue volume. Currently, there are numerous hyaluronic acid hydrogels for dermal fillers available, which can be broadly classified into two categories. The first category of hyaluronic acid hydrogels provides a long-term effect by creating volume and includes fillers such as crosslinked hyaluronic acid (HA) hydrogels. The second category of hyaluronic acid hydrogels provides a long-term effect by inducing neocollagenesis. The best-known and widely used example is Radiesse®, which comprises calcium hydroxyapatite microspheres, a gel carrier of carboxymethyl cellulose (CMC) and glycerin.

Ideal dermal hyaluronic acid hydrogels should be biocompatible, have a low adverse event profile, and provide a reasonably long-lasting persistence (longevity), an effective volumizing capacity and ease of injection. Hyaluronic acid-based hydrogels offer many of these desirable properties of dermal fillers. Since HA is found in almost all species, it has no antigenicity and exhibits excellent tolerance. Furthermore, the crosslinking of hyaluronic acid allows the production of crosslinked hyaluronic acid products that have excellent lifting capacity and are stable for more than 12 months up to two years.

However, since such hyaluronic acid hydrogel is injected into a skin through injection, it accompanies a pain of a patient during the operation or injection. In order to reduce such pain, injections in which local anesthetics such as lidocaine are mixed are used. However, these formulations have a problem in that lidocaine is slowly released. Thus, studies on hyaluronic acid hydrogel formulations capable of performing initial release rapidly and thus reducing the pain of the patient have been pursued.

Given these circumstances, the present inventors have confirmed that when a local anesthetic is applied to an inner wall of a pre-filled syringe in a specific amount and absorbed thereto, and then filled with hyaluronic acid hydrogel, and then adsorbed to the hydrogel by a heating process such as sterilization, the local anesthetic is rapidly released during injection of hyaluronic acid hydrogel, and the anesthetic effect is rapidly exhibited, which can quickly reduce a pain of a patient which may occur during the operation, and further even after the operation, the effect of moisturizing a skin, increasing skin elasticity and improving wrinkles is maintained for a long period of time, thereby completing the present invention.

### [DETAILED DESCRIPTION OF THE INVENTION]

### [Technical Problem]

In order to solve the above problems, it is an object of the present invention to provide a method for producing a hyaluronic acid hydrogel containing a local anesthetic with an increased dissolution rate of the local anesthetic.

### [Technical Solution]

In order to achieve the above object, one embodiment of the present invention provides a hyaluronic acid hydrogel pre-filled syringe comprising a hyaluronic acid hydrogel and a local anesthetic, wherein the local anesthetic is adsorbed on the hydrogel filled in the pre-filled syringe.

The term "pre-filling type syringe" (hereinafter, also referred to as "pre-filled syringe") is a form of an injection that can be used immediately after a pharmaceutical composition is pre-filled in the syringe, and the pre-filled syringe according to the present invention means that a local anesthetic or a pharmaceutically acceptable salt thereof is filled inside the syringe while being adsorbed outside the hyaluronic acid hydrogel. The syringe may include a conventional pre-filled syringe, for example, a barrel, gasket, plunger, cap, or the like, without being limited thereto. Also, the material of the syringe may be a material used as a conventional pre-filled syringe. For example, glass, or a plastic lighter than glass, such as polyester, polycarbonate, polypropylene, cyclic olefin copolymer (COC), cyclic olefin polymer (COP), etc. can be used without limitation. Preferably, it may be a glass or cyclic olefin copolymer (COC).

Further, the volume of the syringe may be appropriately selected as needed, and preferably, it may be 1 to 10 ml, without being limited thereto.

Preferably, the pre-filled syringe according to the present invention can be used for various purposes, but can be used for skin fillers.

As used herein, the term "dermal filler" broadly refers to a material or composition designed to add volume to areas of soft tissue deficiency. The term " dermal filler" as used herein has the same meaning as the term "soft tissue filler" and is used interchangeably therewith. This is, the term "dermal filler" should not be construed as imposing any limitations as to the location and type of injection, and it generally encompasses uses at multiple levels beneath the dermis, for example sub-muscularly above the periosteum and in the subcutaneous plane. Within the meaning of the present invention, the term "soft tissue" generally relates to tissues that connect, support, or surround other structures and organs of the body. In the present invention, the soft tissue includes, for example, muscles, tendons (bands of fibers connecting muscles to bones), fibrous tissues, fats, blood vessels, nerves, and synovial tissues (tissues around joints).

The hyaluronic acid hydrogel, which is one component contained in the pre-filled syringe of the present invention, means a crosslinked hyaluronic acid hydrogel preferably having a crosslinked structure. The crosslinked hyaluronic acid forms a matrix, and such matrix can have a network of crosslinked or non-crosslinked polysaccharides in the form of a solution or a gel. Further, the hyaluronic acid may be hyaluronic acid, hyaluronate, and optional hyaluronate salt, such as sodium hyaluronate, potassium hyaluronate, magnesium hyaluronate, calcium hyaluronate, or a combination thereof.

The hyaluronic acid may be a high molecular weight hyaluronic acid or a low molecular weight hyaluronic acid, and the high molecular weight hyaluronic acid refers to a hyaluronic acid material having a molecular weight of at least about 1.0 million daltons (MW ≥ 10⁶ Da or 1 MDa) to about 4.0 MDa. The low molecular weight hyaluronic acid refers to a hyaluronic acid material having a molecular weight of less than about 1.0 MDa.

Particularly preferably, the hyaluronic acid may have an average molecular weight of 1200, 8,000 to 15,000, 15,000 to 30,000, 30,000 to 50,000, 50,000 to 70,000, 70,000 to 120,000, 90,000 to 110,000, 120,000 to350,000, 130,000 to 150,000, 150,000∼300,000, 300,000 to 500,000, 500,000 to 750,000, 750000 to 1,000,000, 1,000,000 to 1,250,000, 1,250,000 to1,500,000, 1,500,000 to 1,750,000, 1,750,000 to 2,000,000, 2,000,000 to 2,200,000, and 2,000,000 to 2,400,000.

The crosslinked hyaluronic acid includes a crosslinked hyaluronic acid produced from a single hyaluronic acid or from two or more hyaluronic acids having different molecular weights.

The crosslinked hyaluronic acid (HA) generally includes a crosslinked hyaluronic acid (HA) component selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene, 1-(2,3-epoxypropyl)-2,3-epoxycyclohexane, and hexamethylenediamine (HMDA).

Further, preferably, the crosslinked hyaluronic acid hydrogel according to the present invention may be produced with a crosslinking degree of at least about 0.5% to maximum about 50% and an average molecular weight of 1,000,000 to 50,000,000.

Such hyaluronic acid hydrogel may be included in different amounts depending on the volume of the pre-filled syringe to be filled, but preferably, it may be included in an amount of at least 10 mg/mL and up to about 50 mg/ml based on the total volume of the pre-filled syringe.

Additionally, the hyaluronic acid hydrogel may further include one or more selected from the group consisting of cellulose, chitosan, dextran, dextran sulfate, chondroitin, chondroitin sulfate, heparin, heparin sulfate, and alginate. Specifically, one or more selected from the group consisting of cellulose, chitosan, dextran, dextran sulfate, chondroitin, chondroitin sulfate, heparin, heparin sulfate and alginate can be bound to hyaluronic acid constituting the hyaluronic acid hydrogel to form hydrogel. The method for producing such hydrogel can be performed by a known method.

The pre-filled syringe of the present invention is characterized in that a local anesthetic or a pharmaceutically acceptable salt thereof is coated onto the surface of the hyaluronic acid hydrogel filled in the syringe. Also, preferably, the local anesthetic or a pharmaceutically acceptable salt thereof may be lidocaine or a pharmaceutically acceptable salt thereof, without being limited thereto. Lidocaine used in the present invention is a white or yellow crystalline powder of Formula C₁₄H₂₂N₂₀ and is well soluble in alcohol and chloroform. This is an amide-type local anesthetic synthesized by Lofgren and Lund Beast (Sweden) in 1943 and has anesthetic sedative, analgesic and spasmodic actions and also has a cardiac depression action. It acts topically on the skin mucosa and is also used for treating ventricular arrhythmias. The lipophilicity is about 4 times that of procaine, the protein binding capacity is 10 times, the anesthetic power is 4 times and the anesthesia time is 1.5 times. It is widely used in all types of local anesthesia.

However, a local anesthetic may include one or more of various known local anesthetics, as well as such lidocaine or a pharmaceutically acceptable salt thereof, and examples thereof may be selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl paminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and a salt thereof.

Preferably, the pharmaceutically acceptable salt includes a pharmaceutically acceptable inorganic or organic acid. For example, examples of suitable acids include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Preferably, in the present invention, it may be a lidocaine hydrochloride, without being limited thereto.

In the present invention, 0.05 to 1.50% by weight (w/w) of a local anesthetic or a pharmaceutically acceptable salt thereof relative to the weight of the total composition in the pre-filled syringe is coated (adsorbed) onto a hyaluronic acid hydrogel filled in a pre-filled syringe. Preferably, when a local anesthetic is applied to the inner wall of the pre-filled syringe and then the hyaluronic acid hydrogel is filled thereto, the local anesthetic applied to the inner wall is coated and adsorbed onto the surface of the hyaluronic acid hydrogel. In a preferred embodiment, the concentration of a local anesthetic such as lidocaine or a pharmaceutically acceptable salt thereof in the injection may vary depending on the volume of the syringe, but may be included at 0.5 to 15 mg/mL relative to the capacity (volume) of the syringe. The local anesthetic such as lidocaine or a pharmaceutically acceptable salt thereof can be applied by a known coating method such as spraying. When a local anesthetic is applied to the inner wall of the syringe by spraying or the like in this way, unlike the conventional method of mixing a hydrogel such as hyaluronic acid with a local anesthetic, the local anesthetic rapidly acts on the injection or surgery site at the same time as the injection or surgery, and exhibits a rapid anesthetic effect. The preferred concentration of the local anesthetic or a pharmaceutically acceptable salt thereof in the pre-filled injection according to the present invention can be obtained preferably by spraying about 3 to 9 times.

The pre-filled injection according to the present invention may further include a pharmaceutically acceptable additive in addition to the above. Preferably, 94 to 100% of the local anesthetic or a pharmaceutically acceptable salt thereof elutes within 30 minutes of dissolution immediately after production, and 85% to 100% of the local anesthetic or a pharmaceutically acceptable salt thereof elutes within 30 minutes of dissolution even after storage for a long period of time, for example, even after storage at room temperature for 2 months.

The pre-filled injection according to the present invention is administered for treating a cosmetic condition, for example, for treating wrinkles and lines of the skin (e.g., facial lines and facial wrinkles), glabellar lines, nasolabial folds, chin folds, marionette lines, buccal commissures, peri-oral wrinkles, crow's feet, cutaneous depressions, scars, temples, subdermal support of the brows, malar and buccal fat pads, tear troughs, nose, lips, cheeks, perioral region, infraorbital region, facial asymmetries, jawlines, and chin. However, the pre-filled injection according to the present invention may also be administered for treating a therapeutic indication, such as stress urinary incontinence, vesico-ureteral reflux, vocal fold insufficiency, vocal fold medialization.

The pre-filled injection according to the present invention has advantages in that compared to a conventional pre-filled injection filled by mixing a hyaluronic acid and a local anesthetic or a pharmaceutically acceptable salt thereof, it is configured to exhibit an excellent initial dissolution rate of a local anesthetic or a pharmaceutically acceptable salt thereof, and even when distributed and stored for a long period of time, it can rapidly exhibit the anesthetic effect during injection of skin filler or the like.

In another aspect, the present invention relates to a method for manufacturing a pre-filled syringe comprising hyaluronic acid and a local anesthetic or a pharmaceutically acceptable salt thereof, the method comprising the steps of:
(a) spraying a solution containing a local anesthetic or a pharmaceutically acceptable salt thereof on the wall of a syringe to adsorb the local anesthetic or a pharmaceutically acceptable salt thereof and dry it;
(b) filling a hyaluronic acid hydrogel in the syringe adsorbed with the local anesthetic produced in step (a) or a pharmaceutically acceptable salt thereof; and
(c) sterilizing the hyaluronic acid hydrogel filled in a syringe adsorbed with a local anesthetic produced in step (b) or a pharmaceutically acceptable salt thereof.

Specifically, in step (a), a solution containing a local anesthetic or a pharmaceutically acceptable salt thereof such as lidocaine or a pharmaceutically acceptable salt thereof (a spray solution of a local anesthetic or a pharmaceutically acceptable salt thereof) is sprayed on the inner wall of the pre-filled syringe and allowed to adsorb to the inner wall. To spray a local anesthetic or a pharmaceutically acceptable salt thereof, the local anesthetic or a pharmaceutically acceptable salt thereof can be mixed with hyaluronic acid, which can be dissolved using a proper solvent (for example, PBS) to be suitable for being sprayed. The hyaluronic acid used herein is used to ensure that a local anesthetic or a pharmaceutically acceptable salt thereof is uniformly adsorbed onto the inner wall of the syringe, and it may be hyaluronic acid itself, or a salt form of hyaluronic acid, or a crosslinked hyaluronic acid. Preferably, those having an average molecular weight of 1200 to 1,000,000 daltons can be used, and the concentration of hyaluronic acid relative to the lidocaine spray solution is preferably 0.1 to 50.0 (w/w)%.

Preferably, in step (a), when a spray solution of a local anesthetic or a pharmaceutically acceptable salt thereof is sprayed on the inner wall of a syringe, the number of times of spraying may be 3 to 9 times. When spraying beyond the above range, the content of the sprayed lidocaine itself is low and so may be not sufficiently adsorbed on the inner wall of the syringe, or it is sprayed excessively and rather the adsorbed lidocaine solution may be dissolved.

In addition, the concentration of a local anesthetic in the spray solution of a preferred local anesthetic or a pharmaceutically acceptable salt thereof is 1 to 10 (w/w)%, more preferably 10 (w/w)%. When using a spray solution having a concentration of a local anesthetic or a pharmaceutically acceptable salt thereof within the above range, the initial release rate of a local anesthetic or pharmaceutically acceptable salt thereof is significantly increased.

In step (a), a spray solution containing a local anesthetic or a pharmaceutically acceptable salt thereof can be sprayed and adsorbed onto the inner wall of the syringe, and then dried by an appropriate drying method. This drying process can be performed by repeating the process of drying immediately after spraying a spray solution of a local anesthetic or a pharmaceutically acceptable salt thereof once, and then again spraying a spray solution of a local anesthetic or a pharmaceutically acceptable salt thereof on the dried adsorbent layer and then drying it. Preferably, the drying can be performed in a drying oven at a drying temperature of 50 to 90°C, preferably 70°C for about 30 minutes to 2 hours, preferably 1 hour.

Further, in step (b), a hyaluronic acid hydrogel is filled in a syringe adsorbed with a local anesthetic produced in step (a) or a pharmaceutically acceptable salt thereof. The filling method can be performed according to a known filling method.

Further, in step (c), the hyaluronic acid hydrogel that is filled in a syringe adsorbed with a local anesthetic or a pharmaceutically acceptable salt thereof is sterilized and coated (absorbed) onto the hyaluronic acid hydrogel filled with a local anesthetic or a pharmaceutically acceptable salt thereof that has been adsorbed onto the wall. As the sterilization conditions, the sterilization conditions of a known injection may be applied without limitation, and for example, the sterilization can be performed under the conditions of a temperature of 120 to 123°C, a pressure of 1.2 to 1.7 atm and 10 to 20 minutes, preferably 121°C, 1.5 atm and 15 minutes.

In addition, all of those except for the matters not particularly stated in the production method can be applied in the same manner as disclosed in the production method of the pre-filled injection of the present invention.

Yet another embodiment of the present invention provides a method for reducing a pain of a patient when injecting hyaluronic acid hydrogel into a human body, the method comprising the step of injecting a hyaluronic acid hydrogel in the human body by using a hyaluronic acid hydrogel pre-filled syringe comprising hyaluronic acid hydrogel and a local anesthetic, wherein the local anesthetics is adsorbed on the hydrogel filled in the pre-filled syringe. All of those except for the matters not particularly stated in the method for reducing a pain of a patient can be applied in the same manner as disclosed in the production method of the pre-filled injection of the present invention.

### [ADVANTAGEOUS EFFECTS]

The pre-filled injection comprising a local anesthetic or a pharmaceutically acceptable salt thereof and hyaluronic acid wherein the local anesthetic or a pharmaceutically acceptable salt thereof, such as lidocaine or a pharmaceutically acceptable salt thereof, is adsorbed onto an inner wall of a syringe in accordance with the present invention has advantages in that compared to a conventional prefilled injection filled by mixing a hyaluronic acid and a local anesthetic or a pharmaceutically acceptable salt thereof, it is configured to exhibit an excellent initial dissolution rate of a local anesthetic or a pharmaceutically acceptable salt thereof and thus rapidly exhibit the anesthetic effect at the time of injecting a dermal filler.

### [BRIEF DESCRIPTION OF DRAWINGS]

FIG. 1 is a graph comparing the loading rate according to the number of times of repetitive spraying of a lidocaine solution.
FIG. 2 is a graph comparing the dissolution rate according to the number of times of repetitive spraying of a lidocaine solution.
FIG. 3 is a graph showing the dissolution rate of lidocaine dissolved when produced in a mixed type in which lidocaine and hyaluronic acid are mixed and in a spray type (9 times) according to the present invention.
FIG. 4 is a graph showing the dissolution rate of lidocaine when stored at room temperature for a long period of time.

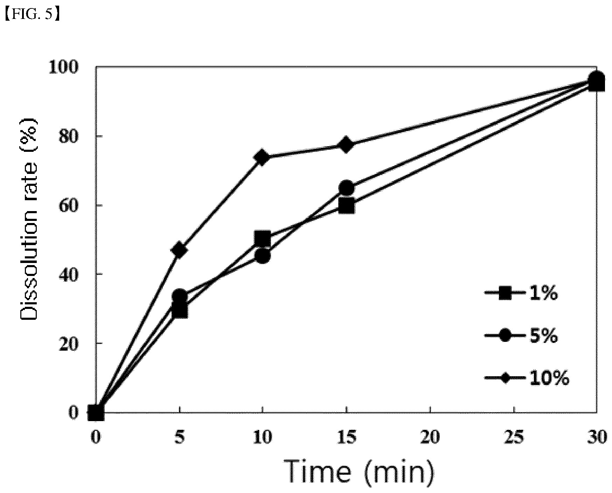

FIG. 5 is a graph showing the dissolution rate according to the concentration of lidocaine in the lidocaine spray solution.

### [DETAILED DESCRIPTION OF THE EMBODIMENTS]

Hereinafter, the present invention will be described in detail by way of examples. However, these examples are for illustrative purposes only, and the present invention is not limited thereto.

### Example 1: Comparative test of the release rate of lidocaine according to the method of adding lidocaine

### 1. Purpose of the experiment

The release rates according to the method of adding lidocaine to the hyaluronic acid (hereinafter referred to as "HA") crosslinked composition were compared to confirm the stability.

### 2. Test substance and preparation

### 2.1 Test substance

**[Table 1]**

| Category | HA concentration (mg/mL) | Lidocaine concentration (%) |
|---|---|---|
| for mixing | 24 | 0.03 |
| for spray | 24 | 10.0 |

### 2.2 Preparation of test substances

Test substances were prepared as follows based on the method for adding lidocaine hydrochloride.

### (1) Gel mixing type

After 0.03 g of lidocaine hydrochloride was dissolved in 100 mL of PBS, 2.4 g of crosslinked hyaluronic acid was added thereto and swelling was performed to produce a hydrogel.

### (2) Spray type in syringe

① 2.4 g of crosslinked hyaluronic acid was put into 100 ml of PBS and swelling was performed to produce a hydrogel.
② 1 g of lidocaine hydrochloride was dissolved in 100 mL of PBS to prepare a lidocaine solution.
③ The lidocaine solution prepared in ① was sprayed 3 times inside a 1 mL pre-filled syringe and dried in a 70°C dry oven for 1 hour. This process was repeated 3 to 9 times.
④ Hyaluronic acid hydrogel was filled in a 1 mL pre-filled syringe prepared in ①.
⑤ Test substance prepared in ④ was sterilized (sterilization condition: 121°C, 1.5 atm, 15 min).

### 3. Test method

### 3.1 Dissolution test

(1) Solution for dissolution test
   - PBS solution (Phosphate-Buffered Saline, IX, pH 7.4 ± 0.1, Welgene)
(2) Temperature for dissolution test: 37.0°C± 0.5°C
(3) Test method
   - About 0.5 mL of gel was taken, put in a 24-well plate and 1 mL of solution for dissolution test was added, and the test was performed in a 37.0°C oven. After the start of the dissolution test, 200 µL of the eluate was taken for every 5, 10, 15, 30, and 60 minutes. Every time the sample solution was collected, 200 µL of the solution for dissolution test previously warmed to 37.0°C ± 0.5°C was added.

### 3.2 Analysis of the dissolution amount of lidocaine

(1) Analytical instrument: Microplate reader (Tecan, Spark 10 M, Austria)
(2) Absorbance: 230 nm
(3) Measurement method: After confirming the standard calibration curve for each concentration, the eluate was analyzed over time and the dissolved amount of lidocaine was calculated.

### 4. Test results

### 4.1 Comparison of the loading rate according to the number of times of repetitive spraying of lidocaine solution.

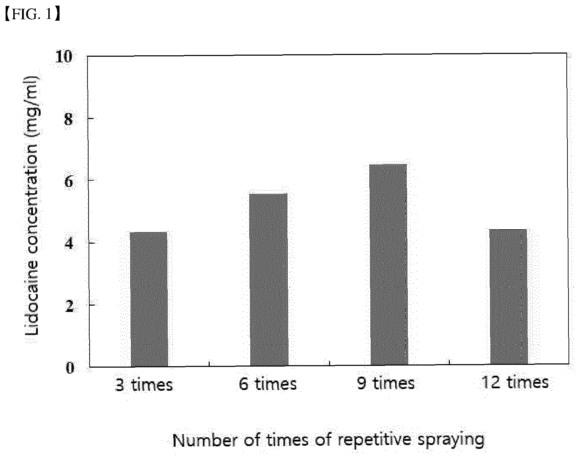

The loading rate of lidocaine according to the number of times of repetitive spraying of lidocaine solution was measured, and the results are shown in FIG. 1. As can be seen in FIG. 1, it was confirmed that in the number of times of repetitive spraying, 9 times is the highest loading rate in the pre-filled syringe. In the case of 9 times or more, it was confirmed that the lidocaine solution adsorbed in the syringe was rather dissolved.

### 4.2 Comparison of Dissolution rate according to the number of times of repetitive spraying

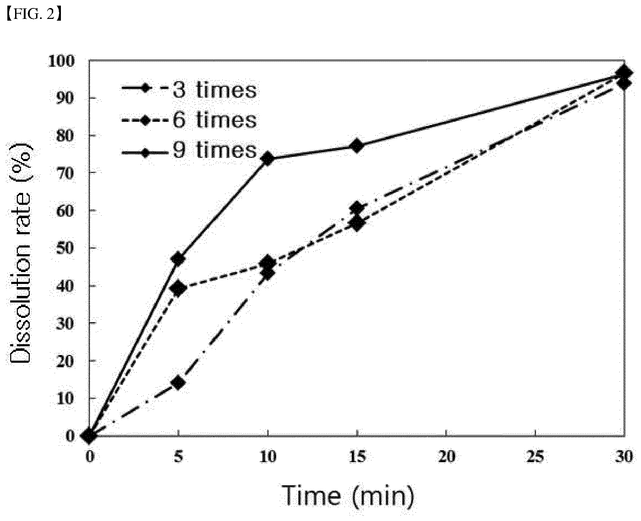

The dissolution rate of lidocaine according to the number of times of repetitive spraying was measured, and the results are shown in FIG. 2. As can be seen in Figure 2, it was confirmed that as the number of times of repetitive spraying increases, the dissolution amount and the initial release rate are faster, and it was also confirmed that lidocaine is released within 10 minutes during 9 sprays.

### 4.3 Comparison of release rate according to the method of adding lidocaine

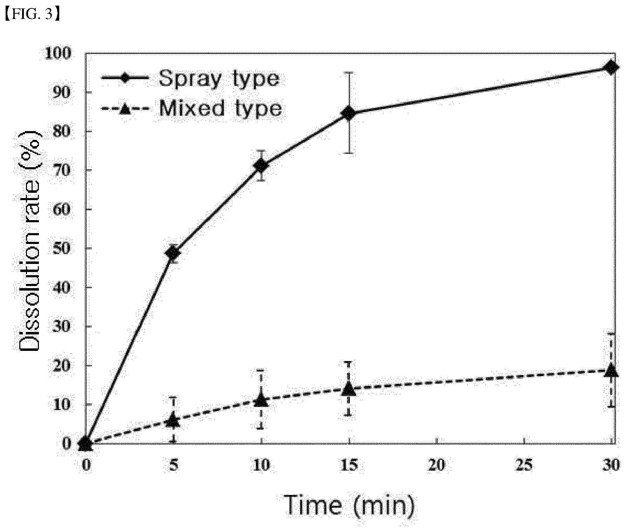

The results of confirming the dissolution rate over time of the pre-filled injection in which lidocaine was adsorbed to the inner wall of the syringe by 9 sprays according to the present invention and the conventional mixed injection prepared by mixing lidocaine and hyaluronic acid are shown in Table 2 and FIG. 3.

**[Table 2] Dissolution rate by hour (%)**

| Time (min) | Spray type (N=3) | Mixed type (N=3) |
|---|---|---|
| 5 | 48.6±2.2 | 6.1±5.6 |
| 10 | 71.12±3.7 | 11.3±7.4 |
| 15 | 84.6±10.3 | 14.1±6.9 |
| 30 | 96.3±0.1 | 18.8±9.4 |

As can be seen in Table 2 and FIG. 3, in the case of a spray-type injection in which a lidocaine solution was sprayed in a syringe, it was confirmed that lidocaine is released within 30 minutes. In the case of the mixed type, it was confirmed that lidocaine in the gel do not escape within 30 minutes and thus, the initial release is very low.

### 4.4 Comparison of dissolution rate according to storage period

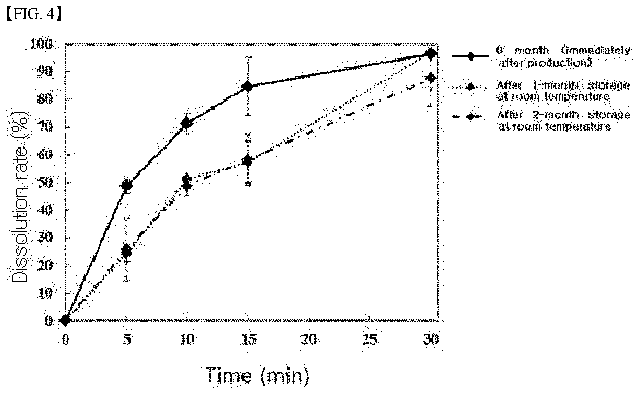

The pre-filled injection of lidocaine adsorbed on the inner wall of the syringe by the 9th spray according to the present invention was stored at room temperature for 2 months, and the dissolution rate after 1 month and 2 months immediately after production was measured, and the results are shown in FIG. 4 and Table 3.

**[Table 3] Comparison of dissolution rate (%)**

| rime (min) | 0 month (immediately after production) (%) | 1-month storage at room temperature (%) | 2-month storage at room temperature (%) |
|---|---|---|---|
| 5 | 48.6±2.3 | 24.4±3.1 | 25.8±11.3 |
| 10 | 71.1±3.7 | 51.2±0.1 | 48.6±3.1 |
| 15 | 84.6±10.3 | 57.1±7.6 | 58.2±9.2 |
| 30 | 96.3±0.2 | 96.8±0.5 | 87.8±10.3 |

As can be seen in Tables 3 and 4, it was confirmed that in the pre-filled composition according to the present invention, the sprayed lidocaine solution is rapidly eluted within 30 minutes even after being stored at room temperature for 1 or 2 months.

### 5. Conclusion

As described above, the release rate according to the method of adding lidocaine to the crosslinked composition was compared. As a result, in the case of the spraying method, the lidocaine solution shows the fastest dissolution rate in the initial stage when it was repeatedly sprayed 9 times, which showed a difference of about 5 times compared to the existing mixed type (30 minutes of Dissolution). In addition, as a result of comparing the dissolution rate according to the storage period, it was confirmed that 85% or more of lidocaine is released within 30 minutes after 2 months at room temperature.

### Example 2: Comparison of loading rate and dissolution rate of lidocaine according to syringe material / syringe volume

In order to compare the loading rate and dissolution rate of lidocaine according to the syringe material/syringe volume, an experiment was conducted according to the following conditions to identify the loading rate and dissolution rate of lidocaine.
1) Syringe material: cyclic olefin copolymer (COC)
2) Syringe volume: 1 mL, 3 mL, 10 mL

### <Dissolution conditions>

- PBS solution, 37□
- Lidocaine solution concentration: 10%
- Method: After 3 sprays in a syringe, dried in a 70 □ drying oven
- Number of times of spray repetitions: based on 9 times

Tables 4 and 5 below show the loading rate and dissolution rate of lidocaine by the volume of the syringe.

**[Table 4] Comparison of loading rate of lidocaine solution by the volume of the syringe**

| Category | Lidocaine concentration (mg/mL) |
|---|---|
| COC 1 mL | 3.23 |
| COC 3 mL | 7.27 |
| COC 10 mL | 11.63 |

**[Table 5] Comparison of dissolution rate**

| Dissolution time (min) | COC 1 mL | COC 3 mL | COC 10 mL |
|---|---|---|---|
| 0 | 0 | 0 | 0 |
| 5 | 11.79% | 26.03% | 8.92% |
| 10 | 49.50% | 51.44% | 37.70% |
| 15 | 63.51% | 79.06% | 66.94% |
| 30 | 96.14% | 94.49% | 99.66% |

Further, under the same conditions as above, a glass syringe with a volume of 3mL was used as a pre-filled syringe, and the dissolution rate of lidocaine was confirmed. The results are shown in Table 6 below.

**[Table 6] Dissolution rate of lidocaine when using a glass pre-filled syringe**

| Category | Dissolution time (min) | Dissolution rate (%) |
|---|---|---|
| 3 mL | 5 | 7.24 |
| | 10 | 37.09 |
| | 15 | 57.01 |
| | 30 | 94.58 |

### Example 3: Confirmation of the effect due to the composition of lidocaine spray solution

In order to confirm whether or not production of an injection according to the hyaluronic acid concentration of the lidocaine spray solution is made possible, a lidocaine spray solution was prepared in PBS according to the following concentration and sprayed, and the results are shown in Table 7 below.

**[Table 7]**

| HA (1,000,000) concentration [(w/v)%] | Lidocaine concentration [(w/v)%] | Whether spraying is made possible |
|---|---|---|
| 0.1 | 10 | Possible |
| 0.5 | | Possible |
| 1.0 | | Spraying is made possible but flow down |
| 3.0 | | Impossible due to high viscosity |
| 5.0 | | Impossible due to high viscosity |

As can be seen in Table 7, when the concentration of hyaluronic acid is 0.1 (w/v)% or more and less than 1.0, it was confirmed that even if the concentration of lidocaine is the same, an injection can be prepared by appropriately spraying.

Subsequently, the loading rate and dissolution rate according to the number of times of spraying of the lidocaine spray solution containing 0.1 and 0.5 (w/w)% of hyaluronic acid showing a desirable lidocaine spray effect were measured. The experimental conditions are as follows. The results are shown in Tables 8 and 9 below.

### <Dissolution conditions>

(1) Temperature: 37 °C
(2) Method: After spraying 3 times in a syringe, dried in a 70 °Cdrying oven

**[Table 8] Comparison of loading rate by the number of times of spraying of 0.5% hyaluronic acid lidocaine spray solution**

| Number of times of spraying | Lidocaine concentration (mg/mL) |
|---|---|
| 3 times | 4.29 |
| 6 times | 4.94 |
| 9 times | 10.04 |

**[Table 9] Comparison of dissolution rate by the number of times of spraying of 0.5% hyaluronic acid lidocaine spray solution**

| HA (1,000,000) concentration (%) | Number of times of spraying | Dissolution time (min) | Dissolution rate (%) |
|---|---|---|---|
| 0.5 | 3 times | 5 | 18.04 |
| | | 10 | 47.64 |
| | | 15 | 64.84 |
| | | 30 | 97.58 |
| | 6 times | 5 | 19.10 |
| | | 10 | 44.12 |
| | | 15 | 65.49 |
| | | 30 | 93.03 |
| | 9 times | 5 | 19.72 |
| | | 10 | 46.65 |
| | | 15 | 63.44 |
| | | 30 | 98.95 |

**[Table 10] Dissolution rate of 0.1% hyaluronic acid lidocaine spray solution**

| HA (1,000,000) concentration [(w/v)%] | Number of times of spraying | Dissolution time (min) | Dissolution rate (%) |
|---|---|---|---|
| 0.1 | 9 times | 5 | 36.29 |
| | | 10 | 59.65 |
| | | 15 | 96.14 |
| | | 30 | 98.13 |

### Example 4: Comparison of loading rate and dissolution rate by lidocaine concentration of lidocaine spray solution

In order to confirm whether or not production of an injection according to the lidocaine concentration of the lidocaine spray solution is made possible, a lidocaine spray solution was prepared in PBS according to the following conditions, and the results are shown in Tables 11 and 12 below.

### <Dissolution conditions>

(1) PBS solution, 37 □
(2) Lidocaine solution concentration: 1 (w/v)%, 5 (w / v)%,
(3) Method: After spraying 3 times in a syringe, dried in a 70□ drying oven
(4) Number of times of repetitive spraying: based on 9 times

**[Table 11] Comparison of loading rates by lidocaine concentration**

| lidocaine concentration [(w/v)%] | lidocaine concentration (mg/mL) | Remarks |
|---|---|---|
| 1% | 0.57 | |
| 5% | 1.08 | |
| 10% | 6.47 | See Example 1 |

As can be seen in Table 11, it was confirmed that as the concentration of the lidocaine spray solution is lower, the loading rate is lower.

**[Table 12] Comparison of dissolution rate**

| Dissolution time (min) | Lidocaine concentration [(w/v)%] | | |
|---|---|---|---|
| | 1 | 5 | 10 |
| 0 | 0 | 0 | 0 |
| 5 | 29.57 | 33.68 | 47.00 |
| 10 | 50.19 | 45.56 | 73.78 |
| 15 | 59.77 | 65.01 | 77.29 |
| 30 | 95.07 | 96.54 | 96.38 |

As can be seen in Table 12, it was confirmed that in the case of the dissolution rate, 95% or more of lidocaine is rapidly released within 30 minutes when spraying at a given concentration. In particular, it was confirmed that the initial release rate is high when the concentration of lidocaine is 5% or more.

### Example 5: Preparation of lidocaine spray solution for each molecular weight of hyaluronic acid

The lidocaine spray solution for each molecular weight of hyaluronic acid was prepared in PBS according to the following concentration and sprayed, and the results were shown in Table 13 below.

**[Table 13]**

| Molecular weight of hyaluronic acid (10,000) | Hyaluronic acid concentration [(w/v)%] | Lidocaine concentration [(w/v)%] | Whether spraying is made possible |
|---|---|---|---|
| 0.12 | 0.1 | 10 | Possible |
| | 0.5 | | Possible |
| | 1.0 | | Possible |
| 0.8 ∼ 1.5 | 0.1 | | Possible |
| | 0.5 | | Possible |
| | 1.0 | | Possible |
| 1.5 ∼ 3.0 | 0.1 | | Possible |
| | 0.5 | | Possible |
| | 1.0 | | Possible |
| 3.0 ∼ 5.0 | 0.1 | | Possible |
| | 0.5 | | Possible |
| | 1.0 | | Possible |
| 5.0 ∼ 7.0 | 0.1 | | Possible |
| | 0.5 | | Possible |
| | 1.0 | | Possible |
| 7.0 ∼ 12.0 | 0.1 | | Possible |
| | 0.5 | | Possible |
| | 1.0 | | Possible |
| 9.0 ∼ 11.0 | 0.1 | | Possible |
| | 0.5 | | Possible |
| | 1.0 | | Possible |
| 12.0 ∼ 35.0 | 0.1 | | Possible |
| | 0.5 | | Possible |
| | 1.0 | | Possible |
| 13.0 ∼ 15.0 | 0.1 | | Possible |
| | 0.5 | | Possible |
| | 1.0 | | Possible |
| 15.0 ∼ 30.0 | 0.1 | | Possible |
| | 0.5 | | Possible |
| | 1.0 | | Possible |
| 30.0 ∼ 50.0 | 0.1 | | Possible |
| | 0.5 | | Possible |
| | 1.0 | | Possible |
| 50.0 ∼ 75.0 | 0.1 | | Possible |
| | 0.5 | | Possible |
| | 1.0 | | Possible |
| 75.0 ∼ 100.0 | 0.1 | | Possible |
| | 0.5 | | Possible |
| | 1.0 | | Possible |

As can be seen in Table 13, when the concentration of hyaluronic acid is 0.1 (w/v)% or more and less than 1.0, it was confirmed that even if the concentration of lidocaine is the same, it can be suitably sprayed at the molecular weight of hyaluronic acid of 1,000,000 or less to prepare an injection. In addition, it was judged that as the molecular weight is lower, the concentration of hyaluronic acid is higher, and thus production is made possible.

### Example 6: Manufacture of pre-filled syringe by type of local anesthetic

A spray solution was prepared with a local anesthetic component or a pharmaceutically acceptable salt thereof, and sprayed on the inner wall of the syringe, and the results are shown in Table 14 below.

**[Table 14]**

| Component name of local anesthetic | Concentration of local anesthetic [(w/v)%] | Whether production is made possible |
|---|---|---|
| Ambucaine | 10 | Possible |
| Amylocaine | | Possible |
| Benzocaine | | Possible |
| Betoxycaine | | Possible |
| Bupivacaine | | Possible |
| Butacaine | | Possible |
| Butanilicaine | | Possible |
| Butoxycaine | | Possible |
| Carticaine | | Possible |
| Chloroprocaine | | Possible |
| Cocaine | | Possible |
| Cyclomethicaine | | Possible |
| Dibucaine | | Possible |
| Dimetocaine | | Possible |
| Etidocaine | | Possible |
| Beta-Eucaine | | Possible |
| Formocaine | | Possible |
| Hexylcaine | | Possible |
| Hydroxytetracaine | | Possible |
| Mepivacaine | | Possible |
| Meprylcaine | | Possible |
| Metabutoxycaine | | Possible |
| Myrtecaine | | Possible |
| Octacaine | | Possible |
| Orthocaine | | Possible |
| Parethoxycaine | | Possible |
| Phenacaine | | Possible |
| Piperocaine | | Possible |
| Pyridocaine | | Possible |
| Prilocaine | | Possible |
| Procaine | | Possible |
| Propanocaine | | Possible |
| Proparacaine | | Possible |
| Propiocaine | | Possible |
| Proxycaine | | Possible |
| Pseudococaine | | Possible |
| Pyrocaine | | Possible |
| Lopivacaine | | Possible |
| Tetracaine | | Possible |
| Tolylcaine | | Possible |
| Trimecaine | | Possible |

As can be seen in Table 14, it was confirmed that an injection can be produced by appropriately spraying with a local anesthetic component or a pharmaceutically acceptable salt thereof.

## Claims

1. A method for manufacturing a pre-filled syringe comprising a local anesthetic or a pharmaceutically acceptable salt thereof and hyaluronic acid hydrogel, the method comprising the steps of: adsorbing a local anesthetic or a pharmaceutically acceptable salt thereof to an inner wall of a pre-filled syringe, filling the hyaluronic acid hydrogel in the syringe, and then coating the local anesthetic or pharmaceutically acceptable salt onto the surface of the hyaluronic acid hydrogel.

2. The method according to claim 1, comprising the steps of:
(a) spraying a solution containing a local anesthetic or a pharmaceutically acceptable salt thereof on the wall of a syringe to adsorb the local anesthetic or a pharmaceutically acceptable salt thereof and dry it;
(b) filling a hyaluronic acid hydrogel in a syringe adsorbed with the local anesthetic produced in step (a) or a pharmaceutically acceptable salt thereof; and
(c) sterilizing the hyaluronic acid hydrogel filled in the syringe adsorbed with a local anesthetic produced in step (b) or a pharmaceutically acceptable salt thereof and absorbing the local anesthetic or a pharmaceutically acceptable salt thereof to the hyaluronic acid hydrogel.

3. The method according to claim 1, wherein the hyaluronic acid hydrogel is a crosslinked hyaluronic acid hydrogel and has a molecular weight of 1,000,000 to 50,000,000 Da.

4. The method according to claim 1, wherein the hyaluronic acid hydrogel further includes one or more selected from the group consisting of cellulose, chitosan, dextran, dextran sulfate, chondroitin, chondroitin sulfate, heparin, heparin sulfate, and alginate.

5. The method according to claim 1, wherein the local anesthetic or a pharmaceutically acceptable salt thereof is one or more selected from the group consisting of lidocaine, ambucaine, amylocaine, benzocaine, betoxycaine, bupivacaine, butacaine, butanilicaine, butoxycaine, carticaine, chloroprocaine, cocaine, cyclomethycaine, dibucaine, dimethocaine, etidocaine, beta-eucaine, formocaine, hexylcaine, hydroxytetracaine, mepivacaine, meprylcaine, metabutoxycaine, myrtecaine, octacaine, orthocaine, parethoxycaine, phenacaine, piperocaine, piridocaine, prilocaine, procaine, propanocaine, proparacaine, propipocaine, propoxycaine, psuedococaine, pyrrocaine, ropivacaine, tetracaine, tolycaine, trimecaine, and a pharmaceutically acceptable salt thereof.

6. The method according to claim 1, wherein the pharmaceutically acceptable salt of the local anesthetic is a lidocaine hydrochloride.

7. The method according to claim 2, wherein the solution containing a local anesthetic or a pharmaceutically acceptable salt thereof in step (a) includes a solution containing a local anesthetic or a pharmaceutically acceptable salt thereof and a solution containing hyaluronic acid.

8. The method according to claim 7, wherein a concentration of hyaluronic acid to the solution containing the local anesthetic or a pharmaceutically acceptable salt thereof is 0.1 to 50(w/w)%.

9. The method according to claim 2, wherein the spaying and drying in step (a) are repeated 3 times to 9 times.

10. The method according to claim 2, wherein the drying of step (1) is performed at 50 to 90°C for 30 minutes to 2 hours.

11. The method according to claim 1, wherein an average molecular weight of hyaluronic acid in the solution containing a local anesthetic or a pharmaceutically acceptable salt thereof in step (a) is 1200 to 1,000,000 Da.

12. The method according to claim 2, wherein the sterilization of step (c) is performed under the conditions of a temperature of 120 to 123°C, a pressure of 1.2 to 1.7 atm and 10 to 20 minutes.

13. A pre-filled syringe filled with a filler composition comprising a local anesthetic or a pharmaceutically acceptable salt thereof and hyaluronic acid hydrogel, wherein the local anesthetic or a pharmaceutically acceptable salt thereof is coated onto the surface of hyaluronic acid hydrogel.

14. The pre-filled syringe according to claim 13, wherein the hyaluronic acid hydrogel is a crosslinked hyaluronic acid hydrogel and has a molecular weight of 1,000,000 to 50,000,000 Da.

15. The pre-filled syringe according to claim 13, wherein the syringe includes one or more selected from the group consisting of glass, or polyester, polycarbonate, polypropylene, cyclic olefin copolymer and cyclic olefin polymer.

16. The pre-filled syringe according to claim 13, wherein the local anesthetic or a pharmaceutically acceptable salt thereof in the syringe is included at 0.5 to 15 mg/mL relative to the volume of the syringe.

17. The pre-filled syringe according to claim 13, wherein the local anesthetic or a pharmaceutically acceptable salt thereof is one or more selected from the group consisting of lidocaine, ambucaine, amylocaine, benzocaine, betoxycaine, bupivacaine, butacaine, butanilicaine, butoxycaine, carticaine, chloroprocaine, cocaine, cyclomethycaine, dibucaine, dimethocaine, etidocaine, beta-eucaine, formocaine, hexylcaine, hydroxytetracaine, mepivacaine, meprylcaine, metabutoxycaine, myrtecaine, octacaine, orthocaine, parethoxycaine, phenacaine, piperocaine, piridocaine, prilocaine, procaine, propanocaine, proparacaine, propipocaine, propoxycaine, psuedococaine, pyrrocaine, ropivacaine, tetracaine, tolycaine, trimecaine, and a pharmaceutically acceptable salt thereof.

18. The pre-filled syringe according to claim 13, wherein the pharmaceutically acceptable salt of the local anesthetic is a lidocaine hydrochloride.

19. The pre-filled syringe according to claim 13, wherein the syringe is for dermal fillers.

20. The pre-filled syringe according to claim 13, wherein the hyaluronic acid hydrogel further includes one or more selected from the group consisting of cellulose, chitosan, dextran, dextran sulfate, chondroitin, chondroitin sulfate, heparin, heparin sulfate, and alginate.

21. A method for reducing a pain of a patient when injecting hyaluronic acid hydrogel, the method comprising the step of injecting a hyaluronic acid hydrogel in a human body by using a hyaluronic acid hydrogel pre-filled syringe comprising hyaluronic acid hydrogel and a local anesthetic, wherein the local anesthetic is coated onto the surface of the hydrogel e filled in the pre-filled syringe.
